# EUROPÄISCHE PATENTSCHRIFT

(11) **EP 1 246 631 B1**
(45) Veröffentlichungstag und Bekanntmachung des Hinweises auf die Patenterteilung: **14.05.2003**
(21) Anmeldenummer: 00908924.4
(22) Anmeldetag: 14.01.2000
(51) Int. Cl.: A61K 35/16, A61K 31/14, A61M 1/36

(54) **VERFAHREN UND VORRICHTUNG ZUR ERHÖHUNG UND/ODER VERRINGERUNG DER KONZENTRATION IMMUNMODULATORISCH WIRKSAMER STOFFE IN STOFFGEMISCHEN**
METHOD AND DEVICE FOR INCREASING AND/OR DECREASING THE CONCENTRATION OF IMMUNOMODULATORY-ACTIVE SUBSTANCES IN SUBSTANCE MIXTURES
PROCEDE ET DISPOSITIF POUR AUGMENTER ET/OU REDUIRE LA CONCENTRATION EN SUBSTANCES A EFFET IMMUNOMODULATEUR DANS DES MELANGES DE SUBSTANCES

(43) Veröffentlichungstag der Anmeldung: 09.10.2002
(73) Patentinhaber: TERAKLIN Aktiengesellschaft, 18119 Rostock-Warnemünde (DE)
(72) Erfinder: ALTRICHTER, Jens, D-18196 Kavelstorf (DE); FREYTAG, Jens, D-18055 Rostock (DE); MITZNER, Steffen, D-18055 Rostock (DE); STANGE, Jan, D-18057 Rostock (DE)
(74) Vertreter: Weber, Dieter, Dr.
(86) Internationale Anmeldenummer: DE0000130
(87) Internationale Veröffentlichungsnummer: WO01051068

(56) Entgegenhaltungen:
- WO-A-93/16171
- DE-A- 19 831 873
- US-A- 5 022 988
- DATABASE BIOSIS [Online] BIOSCIENCES INFORMATION SERVICE, PHILADELPHIA, PA, US; 1999 MITZNER S ET AL: "Cell-based therapy of severe infections: In-vitro and in-vivo results." Database accession no. PREV199900293934 XP000938292 & EUROPEAN JOURNAL OF CELL BIOLOGY, Bd. 78, Nr. SUPPL. 49, 1999, Seite 96 23rd Annual Meeting of the German Society for Cell Biology;Rostock, Germany; March 14-18, 1999 ISSN: 0171-9335
- DATABASE BIOSIS [Online] BIOSCIENCES INFORMATION SERVICE, PHILADELPHIA, PA, US; 1999 KOBALL S ET AL: "Leucocytes as biosensors for biocompatibility investigations of peritoneal dialysate solutions." Database accession no. PREV199900289431 XP000938291 & EUROPEAN JOURNAL OF CELL BIOLOGY, Bd. 78, Nr. SUPPL. 49, 1999, Seite 80 23rd Annual Meeting of the German Society for Cell Biology;Rostock, Germany; March 14-18, 1999 ISSN: 0171-9335
- STANGE J ET AL: "Molecular adsorbent recycling system (MARS): Clinical results of a new membrane-based blood purification system for bioartificial liver support." ARTIFICIAL ORGANS, Bd. 23, Nr. 4, April 1999 (1999-04), Seiten 319-330, XP000949229 ISSN: 0160-564X
- WEISS MANFRED ET AL: "Granulocyte colony-stimulating factor to prevent the progression of systemic nonresponsiveness in systemic inflammatory response syndrome and sepsis." BLOOD, Bd. 93, Nr. 2, 15. Januar 1999 (1999-01-15), Seiten 425-439, XP002147989 ISSN: 0006-4971 in der Anmeldung erwähnt

## Beschreibung

Die Erfindung betrifft ein Verfahren zur Erhöhung und / oder Verringerung der Konzentration immunmodulatorisch wirksamer Stoffe in Stoffgemischen, die potentiell immunmodulatorisch wirksame Stoffe enthalten, sowie eine entsprechende Vorrichtung zur Durchführung des Verfahrens.

### Stand der Technik

Das Immunsystem und seine Modulierung sind in den vergangenen Jahren zunehmend in das Blickfeld der Grundlagen- und angewandten Forschung im Bereich der Biotechnologie, Kosmetik und Medizin, aber auch der Lebensmittel- und Umwelttechnologie getreten. Die Ursachen hierfür liegen zum einen in dem Auftreten neuer und der Ausbreitung bekannter Infektionen, vor allem aber auch dem zunehmenden Auftreten von Substanzen mit immunmodulatorischem Potential, wie z.B. Stoffen in Nahrungsmitteln, Kosmetika oder der Umwelt, die das Immunsystem inadäquat in seiner Funktion verändem und dadurch z.B. Allergien oder Autoimmunerkrankungen auslösen.

### Infektionen

Die schwerste Form einer Infektion, bei der sich die Krankheitserreger über den ganzen Körper ausgebreitet haben, wird als Sepsis bezeichnet. Im Verlauf einer Sepsis treten eine Vielzahl von Veränderungen in der Aktivität der Komponenten des Immunsystems auf, was im Falle des Überschreitens kritischer Grenzen mit dem Tod des Organismus endet.
Das heutige Wissen über den Verlauf und die Pathogenese einer Sepsis stammt wesentlich aus Untersuchungen über die Wechselwirkungen zwischen Gram-negativen Bakterien und dem menschlichen Organismus (Chest 1992; 101; 1644-1655). Die Hauptagenzien der Einleitung einer Sepsiskaskade stellen demnach die bakteriellen Endotoxine, eine Gruppe von Lipopolysacchariden aus der Zellwand Gram-negativer Bakterien, dar (Reviews Infect Dis. 1983; 5; 733-747). Endotoxine, die wahrscheinlich potentesten fiebererzeugenden Substanzen (Pyrogene) überhaupt, aktivieren vor allem Monozyten und Endothelzellen. Durch die Freisetzung von Mediatoren bzw. Ausbildung von Adhäsionsmotekülen wird das Immunsystem aktiviert, ein Leukozytensticking vorbereitet. Es kommt zur Migration der Leukozyten in Gewebe mit hohem Chemotaxingehalt (Orte lokaler Entzündung). Kann die lokale Ursache beseitigt werden, dann sistiert der Entzündungsprozeß. Kommt es über längere Zeit intermittierend oder kontinuierlich zum übermäßigen Einschwemmen von Bakterien, Endotoxinen oder anderen antigen wirkenden Zellprodukten ins Blut, schlägt die sinnvolle Abwehrreaktion von Monozyten und Endothelzellen in einen autoaggressiven Prozeß mit schwersten Kreislaufdysregulationen, sekundären Organversagen, Gerinnungsstörungen (DIC) etc. um: eine Sepsis (mit positivem Erregemachweis) bzw. ein Systemic Inflammatory Response Syndrome (SIRS, kein Erreger nachweisbar) entwickeln sich und führen bei einfacher Sepsis in bis zu 30% der Fälle und im septischen Schock sogar bei bis zu 90 % der Patienten zum Tod (Sepsis. An interdisciplinary challenge. Berlin, Heidelberg, New York: Springer Verlag, 1989).

Gram-positive Erreger als Ursache für Sepsis sind in den letzten Jahren verstärkt in das Blickfeld der Forschung gerückt. Zahlreiche Arbeiten beschäftigen sich mit der steigenden Inzidenz der Gram-positiven Sepsis im letzten Jahrzehnt. Statistiken zeigen, daß bereits jetzt 30 bis 40 % aller Fälle von Sepsis auf Gram-positive Erreger zurückzuführen sind (Am J Med. 1991; 91 (suppl 3B): 72-89). Die Behandlung der bakteriellen Sepsis in den intensivmedizinischen Zentren wird auf Grund der zunehmenden Antibiotikaresistenz zusätzlich erschwert. Gegenwärtig wird die Sepsis als mehrphasiges Krankheitsbild betrachtet, bei dem sich an die Phase der Keimeinschwemmung eine sogenannte Hyperinflammationsphase mit einer übersteigerten Ausschüttung pro-inflammatorischer Zytokine, wie z.B. an Tumor-Nekrose-Faktor alpha (TNF alpha) oder einigen Interleukinen (z.B. Interleukin-1 und Interleukin-6), anschließt. Im weiteren Verlauf kommt es auf Grund von negativen Rückkopplungsmechanismen zu einem überschießenden Umschlag zugunsten der anti-inflammatorischen Zytokine (Transformierender Wachstumsfaktor beta =TGF beta, Interleukin-4, Interleukin-10, Interleukin-13) und damit zur sogenannten Immunparalyse-Phase, welche schließlich zum Tod des Patienten führt (Internist 1997; 38; 541-552). Eine klare Definition der einzelnen Phasen auf Grundlage konkreter paraklinischer Werte ist bisher jedoch noch nicht erfolgt.

Die ursächliche Behandlung der bakteriellen Sepsis ist bis heute nur in Ansätzen möglich. Die Hoffnungen stützten sich neben der Antibiotikatherapie auf die Anwendung antiinflammatorischer Stoffe, die derzeit auf ihre Effekte bei Gram-negativer Sepsis überprüft werden (Nature. 1990; 348: 550-552, FASEB J. 1991; 5: 338-343). Einige Studien hierzu sind bereits abgeschlossen und haben bisher eher enttäuschende Resultate erbracht. So konnten weder gegen Endotoxin-LipidA gerichtete Antikörper (N. Engl. J. Med. 1991; 324; 429-436, JAMA 1991; 266; 1097-1102) noch anti-Zytokin-Therapien gegen Tumor-Nekrose-Faktor alpha (Crit Care Med. 1993; 21; 318-327, JAMA 1995; 273; 934-941) bzw. Interleukin-1 (JAMA 1994; 271; 1836-1842) zu einer Senkung der Gesamtmortalität beitragen. Es scheinen jedoch Patienten mit sehr hohen Zytokinspiegeln zumindestens teilweise zu profitieren (Crit Care Med. 1993; 21; 318-327, Crit. Care Med. 1996; 24; 733-742). Die Studien sind jedoch durch eine große Heterogenität der Patientengruppen sowie die bisher nur unzureichende klinische Stadieneinteilung des Sepsisverlaufs gekennzeichnet.
Ein neuer Ansatz ist der Einsatz pro-inflammatorischer Zytokine (Interferon gamma) in der Phase der Immunparalyse, der in ersten nicht-randomisierten Studien erfolgversprechend scheint, jedoch bisher noch eine Sterblichkeit von über 30 % zeigte (Nature Medicine 1997; 3; 678-681). Patentrechlich angemeldete immunmodulatorische Ansätze sind insbesondere die Gabe von einzelnen immunmodulatorischen Substanzen, wie z.B. einigen oben beschriebene Zytokinantagonisten (WO 94/06431 A1, US 5,585,486, US 5,585,357, US 5,565,430, US 5,552,400), Mistel-Lektine (DE 4221836), Fosfomycin (JP 09183730 A), Macrocyclische Substanzen (US 5,527,907, US 5,541,189, US 5,541,193, US 5,561,139, US 5,561,140 oder bakterielle Extrakte (WO 89/09607, EP 0 363 491). Keiner dieser Ansätze konnte entscheidende therapeutische Gewinne bei der Therapie der Sepsis zeigen.

Ein interessanter Ansatz ist der Einsatz von granulozyten- bzw. makrophagenstimulierenden Faktoren (G-CSF, GM-CSF). Der Verlauf schwerer Septitiden konnte durch die Gabe solcher Faktoren günstig beeinflußt werden (Blood 1999; 93: 425-439; Curr Opin Hematol 1999; 6: 176-183; J Infect Dis 1999; 179: S342-352; Arch Pediatr Adolesc Med 1999; 153: 984-988). Der günstige klinische Effekt wird am ehesten durch eine Steigerung des Differenzierungsgrades und der spezifischen Funktionalität weißer Blutzellen (Granulozyten und Makrophagen) erreicht.

### Allergien

Allergien bezeichnen die durch einen vorangegangene Sensibilisierung ausgelösten Überreaktionen des Immunsystems gegenüber einem üblicherweise nicht körpereigenen Stoff. Sie werden in der Regel durch kleine chemische Gruppen (Haptene), die mit einer größeren chemischen Struktur verbunden sind (dem Haptencarrier, der Gesamtkomplex wird als Vollallergen bezeichnet) ausgelöst. Es werden unterschiedliche Typen allergischer Reaktionen unterschieden. Inbesondere der Typ I ist durch eine vermehrte Bildung von Antikörpern des Immunglobulin E Typs, die gegen das Hapten/Allergen gerichtet sind, vermittelt. Die Bindung und Vemetzung der an oberflächenständige Rezeptoren gebundenen Immunglobulin E Moleküle durch die Allergene führt zur Aktivierung der Immunzellen (vorwiegend Mastzellen und basophile Granulozyten) mit nachfolgender Bildung und Freisetzung einer Reihe Immunmodulatorischer Moleküle (z.B. Histamin, Prostaglandine, Leukotriene und Zytokine wie TNFalpha oder verschiedene Interleukine) (Bundschuh, G, Lexikon der Immunologie, 2. Aufl. Medical Service, München 1992). Gegenwärtige immunmodulatorische Therapien in der Allergiebehandlung konzentrieren sich vor allem auf die Blockade der Freisetzung der immunmodulierenden Substanzen durch Immunsuppressiva (z.B. Glukokortikoide) bzw. auf die Hemmung der Bindung der Substanzen an den Zielzellen (z.B. Histamin- oder Leukotrienrezeptorantagonisten). Eine andere Therapieform ist Verabreichung kleiner mengen des Allergens und langsame Dosissteigerung im Sinne einer Desensibilisierung (Bundschuh, G, Lexikon der Immunologie, 2. Aufl. Medical Service, München 1992).

### Extrakorporale Immunmodulation

Extrakorporale Ansätze im Sinne einer Blutwäsche beinhalteten bisher den Gebrauch von chemischen Adsorbem zum Abreichern von Keimbestandteilen (z.B. DEAE-Cellulose, Polyethylenimin oder PMX-Fiber als Endotoxinadsorber (Mitzner et al. Artificial Organs 1992, 17, 775-781, Samtleben et al. Artificial Organs 1998, 22, 43-46; Tani et al. Artificial Organs 1998, 22, 1038-1044).

Die bisher bekannten immunmodulatorischen Ansätze wurden vorwiegend durch in-vivo Gabe von Substanzen durchgeführt. Bei diesen Substanzen handelt es sich z.B. um die Antibiotika, die die Keimvermehrung verhindern und damit die Keime als immunmodulatorisches Agenz ausschalten. Neuere Ansätze der Immunmodulation wurden mit gegen Zytokine gerichteten Antikörpern beziehungsweise anderen die Zytokine bindenden Eiweißpräparaten durchgeführt und haben keinen bzw. nur geringen therapeutischen Nutzen gebracht. (Internist 1997; 38: 541-552). Endotoxinadsorber haben bisher in-vitro gute Abreicherungen gezeigt. In der extrakorporalen Therapie schwerer Infektionen wurden Endotoxinadsorber bisher nicht in kontrollierten, randomisierten Studien eingesetzt. Eine nichtrandomisierte Studie mit PMX-Fibern stellte jedoch auch in-vivo eine Senkung der Todesrate auf nur noch 46 % fest, was eine immer noch eine unbefriedigend hohe Zahl ist. (Tani et al. Artificial Organs 1998, 22, 1038-1044)

Bei Zuständen der Hyperinflammation bzw. Immunparalyse kommt es zum Anstieg der Blutplasmakonzentrationen einer Vielzahl von immunmodulierenden Stoffen wie z.B. Zytokinen, Keimbestandteilen und Keimprodukten, welche die Vorgänge der Abwehr von Infektionen z.T. erheblich stören. Die Auslöschung bzw. Zugabe eines einzelnen Zytokins hat bisher keinen überzeugenden therapeutischen Erfolg erbracht. Vielmehr sind die sinnvolle Entfernung von zu hohen Konzentrationen einzelner Stoffe sowie die Substitution anderer Stoffe, welche in zu geringer Konzentration vorhanden sind, gleichzeitig gefragt, um das aus der Bahn gebrachte Immunsystem zu stabilisieren. Die Komplexität des Problems der wechselnden Konzentrationen der immunmodulierenden Stoffe erfordert einerseits die sensible Messung, andererseits aber auch die Fähigkeit der schnellen Substitution der jeweiligen Stoffe. Allein durch die Zeitverzögerung bei der Bestimmung der Zytokin-Konzentration werden Substitutionstherapien in Form von Injektionen/Infusionen zukünftig nur bedingt zur Lösung beitragen können.

Die Aufgabe der vorliegenden Erfindung besteht daher darin, ein gegenüber dem Stand der Technik verbessertes Verfahren und eine Vorrichtung zur Veränderung der Konzentration immunmodulatorisch wirksamer Stoffe in einem Stoffgemisch oder einer Lösung bereitzustellen.

Erfindungsgemäß wird diese Aufgabe durch ein Verfahren der eingangs genannten Art gelöst, bei dem man das Stoffgemisch oder die Lösung in einen Bioreaktor einführt und dort mit Zellen in Berührung bringt, wobei die Zellen so ausgewählt sind, dass sie Rezeptoren für wenigstens eine Spezies aus einer Gruppe G1 immunmodulatorisch wirksamer Stoffe, deren Konzentration in dem Stoffgemisch verringert werden soll, aufweisen und in der Lage sind, diese Spezies zu adsorbieren, und / oder dass die Zellen wenigstens eine Spezies aus einer anderen Gruppe G2 immunmodulatorischer Stoffe, deren Konzentration in dem Stoffgemisch erhöht werden soll, herstellen und in der Lage sind, diese Spezies in das Stoffgemisch freizusetzen, und das Stoffgemisch anschließend von den Zellen trennt und aus dem Bioreaktor entfernt.

Weiterhin wird die erfindungsgemäße Aufgabe durch eine Vorrichtung zur Erhöhung und / oder Verringerung der Konzentration immunmodulatorisch wirksamer Stoffe in einem Stoffgemisch gelöst mit einem Bioreaktor mit einem Einlaß zum Einführen des Stoffgemischs und einem Auslaß zur Entnahme des Stoffgemischs, wobei in dem Bioreaktor lebende Zellen in einer Form vorhanden sind, dass das Stoffgemisch davon mit den Zellen in Berührung gebracht werden kann, und mit einer Zellrückhalteeinrichtung, die so ausgelegt ist, dass das Stoffgemisch von den Zellen trennbar und frei von Zellen aus dem Bioreaktor entnehmbar ist, wobei die Zellen Rezeptoren für wenigstens eine Spezies aus einer Gruppe G1 immunmodulatorisch wirksamer Stoffe, deren Konzentration in dem Stoffgemisch verringert werden soll, aufweisen und in der Lage sind, diese Spezies zu adsorbieren, und /oder dass die Zellen wenigstens eine Spezies aus einer anderen Gruppe G2 immunmodulatorischer Stoffe, deren Konzentration in dem Stoffgemisch erhöht werden soll, herstellen und in der Lage sind, diese Spezies in das Stoffgemisch freizusetzen.

Vorzugsweise ist das bei dem erfindungsgemäßen Verfahren eingesetzte Stoffgemisch Blut,. Blutplasma, Blutplasmabestandteile und / oder Wasser einzeln oder in Kombination. Es enthält gegebenenfalls zusätzlich Eiweiße, Peptide, Kohlenhydrate, Lipide, Nukleinsäuren, Salze und / oder Mikroorganismen einzeln oder in Kombination.

Der Einsatz von Zellen, die mit ihren spezifischen Oberflächenrezeptoren immunmodulierende Stoffe selektiv adsorbieren und damit dem Stoffgemisch entziehen, andererseits aber auch selbst die in dem Stoffgemisch unterrepräsentierten immunmodulatorisch wirksamen Moleküle, wie z. B. Zytokine, bilden und freisetzen können, stellt einen neuen Ansatz dar, Stoffgemischen, wie Blut oder Blutplasma eines Patienten, extrakorporal immunologisch in positiver oder negativer Weise aktive Stoffe unter kontrollierten Bedingungen zu entziehen und zuzuführen. Das Stoffgemisch kann das Blut oder ein Blutbestandteil eines Patienten mit einer Infektion, einer Allergie oder einer anderen Erkrankung, die mit einer Veränderung der Konzentrationen immunmodulatorisch wirksamer Stoffe gegenüber dem gesunden Zustand einhergeht, sein. Dem Patienten kann das Stoffgemisch nach der Durchführung des erfindungsgemäßen Verfahrens wieder verabreicht werden. Das Stoffgemisch kann jedoch auch nicht aus demselben Patienten stammen, sondern beispielsweise aus dem Blut eines anderen Individuums, wie eine Blutkonserve, oder es kann eine synthetisch hergestellte Lösung sein, die mittels des erfindungsgemäßen Verfahrens mit immunmodulatorisch wirksamen Stoffen angereichert und anschließend einem Patienten verabreicht wird. Durch die vorliegende Erfindung wird es erstmals möglich, eine komplexe, "intelligente" biologische Modifikation immunologischer Prozesse vorzunehmen und diese gezielt zu steuern.

Das erfindungsgemäße Verfahren erlaubt die Arbeit unter physiologischen Bedingungen (Temperatur, pH-Werte, lonenkonzentrationen, Puffer etc.), was erhebliche Vorteile bei der Prozessierung thermolabiler und anderweitig empfindlicher physiologischer Stoffgemische ermöglicht. Das erfindungsgemäße Zellsystem kann gut auf Stabilität der morphologischen und funktionellen Eigenschaften überwacht werden. Dies ist aus Sicht einer angestrebten größtmöglichen Sicherheit und Selektivität der ablaufenden Prozesse von besonderer Bedeutung. Auch und gerade für eine industrielle Anwendung ist die genaue Beschreibbarkeit ablaufender Operationen von großer Bedeutung und z.B. bei Zulassungsund Qualitätsstandardfragen unerlässlich.

Die hierin beschriebenen Gruppen immunmodulatorisch wirksamer Stoffe, G1 für adsorbierte Stoffe und G2 für freigesetzte oder sezemierte Stoffe, sind nicht so zu verstehen, dass sie voneinander verschieden sein müssen oder zumindest keine gleichen Stoffe enthalten können. Vielmehr ist es so, dass häufig die jeweilige Konzentration eines Stoffes entscheidend ist, ob dieser Stoff in immunologischer Hinsicht positiv oder negativ wirkt, d. h. ob er in dem jeweiligen Stoffgemisch adsorbiert oder freigesetzt werden soll. Bei dem erfindungsgemäßen Verfahren können auch gleiche Stoffe in beiden Gruppen G1 und G2 enthalten sein, wenn die verwendeten Zellen diese sowohl adsorbieren als auch freisetzen und dabei ein Konzentrationsgleichgewicht herstellen. Unter dem Begriff "Stoffe", wie er im Sinne dieser Erfindung gebraucht wird, sind u. a. auch lebende Organismen, wie Bakterien, Pilze, Hefen usw., zu verstehen, die immunmodulatorisch wirksam sind.

Erfindungsgemäß vorteilhaft ist es, wenn die Gruppen G1 und / oder G2 immunmodulatorischer Stoffe Zytokine, vorzugsweise pro-inflammatorische oder anti-inflammatorische Zytokine, lösliche Zytokinrezeptoren, Zytokinrezeptorantagonisten, Wachstumsfaktoren, lösliche Adhäsionsmoleküle, Komponenten oder Spaltprodukte des Gerinnungs-, Fibrinolyse- oder Komplementsystems und reaktive Sauerstoffspezies umfassen.

Besonders bevorzugt umfassen die Gruppen G1 und / oder G2 die bekanntermaßen immunmodulatorisch wirksamen Stoffe Interferon Alpha, Interferon Beta, Interferon Gamma, Interleukin 1, Interleukin 2, Interleukin 3, Interleukin 4, Interleukin 5, Interleukin 6, Interleukin 7, Interleukin 8, Interleukin 9, Interleukin 10, Interleukin 11,, Interleukin 12, Interleukin 13, Interleukin 14, Interleukin 15, Interieukin 16, Interleukin 17, Interleukin 18, Tumor-Nekrose-Faktor Alpha (TNF-alpha), Tumor-Nekrose-Faktor Beta (TNF-beta), Transformierenden Wachstumsfaktor Beta (TGF-beta), Chemokine und Chemotaxine. Weiterhin besonders geeignet sind Endotoxine, vorzugsweise Lipopolysaccharide, Exotoxine, vorzugsweise Hämolysin A, B, C, D oder E, Lipoteicholsäure und Zymosan. Ebenfalls geeignet sind Immunkomplexe, bestehend aus wenigstens einem Antikörper, vorzugsweise einem Immunglobulin, oder einem Antikörperanteil und wenigstens einem Antigen oder antigen wirkendem Stoff, wie einem Hapten.

Erfindungsgemäß können die Gruppen G1 und / oder G2 auch Bestandteile oder Produkte von Mikroorganismen, vorzugsweise Bakterien, Viren, Pilzen oder Parasiten, oder vollständige Mikroorganismen oder allergen wirkende Substanzen umfassen.

Für das erfindungsgemäße Verfahren hat es sich als besonders vorteilhaft erwiesen, wenn die Zellen in dem Bioreaktor Leukozyten, hämatopoietische Stammzellen, aus hämatopoietischen Stammzellen durch Differenzierung gewonnene Zellen, Hepatozyten, Endothelzellen, Nervenzellen, Mukosazellen, Epithelzellen oder andere dem Ektoderm, Mesoderm oder Endoderm entstammende Zellen oder eine Kombination davon sind. Besonders geeignet sind Primärzellen, immortalisierte oder Tumorzellen. Obwohl Zellen jeden Ursprungs in dem erfindungsgemäßen Verfahren eingesetzt werden können, sind Zellen humanen, tierischen, planzlichen oder mikrobiellen Ursprungs besonders geeignet.

Bei einer besonders bevorzugten Ausführungsform der Erfindung werden die Zellen in dem Bioreaktor vor oder während der Einführung und Berührung mit dem Stoffgemisch stimuliert. Durch die Verwendung von spezifisch vorstimulierten ("trainierten") Immunzellen kann eine Störung im immunologischen Gleichgewicht einerseits durch die als multiparametrischer Biosensor fungierende Zelle erkannt werden und gleichzeitig kann diese Störung, komplett oder teilweise, ausgeglichen werden.

Um eine möglichst hohe Vitalität der verwendeten Zellen zu erzielen und die Stoffwechselaktivität zu beeinflussen, ist es zweckmäßig, wenn man Temperatur, Begasung und Nährstoffzufuhr der Zellen in dem Bioreaktor in bekannter Weise reguliert. Als Zellrückhalteeinrichtung, mit der man das Stoffgemisch in dem Bioreaktor nach der Berührung mit den Zellen trennt eignet sich ein Zellfilter oder eine Zentrifuge.

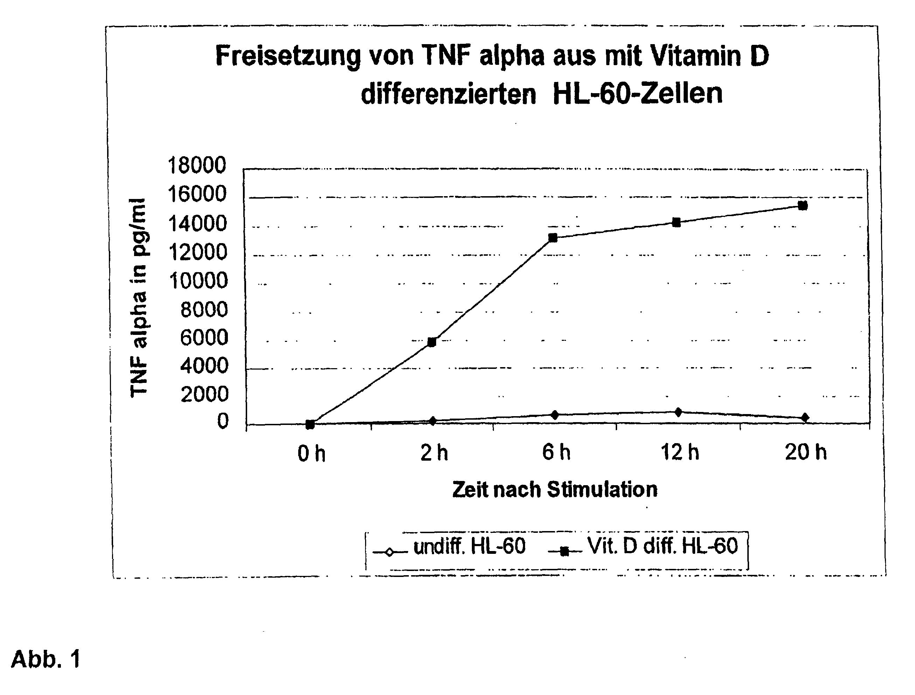

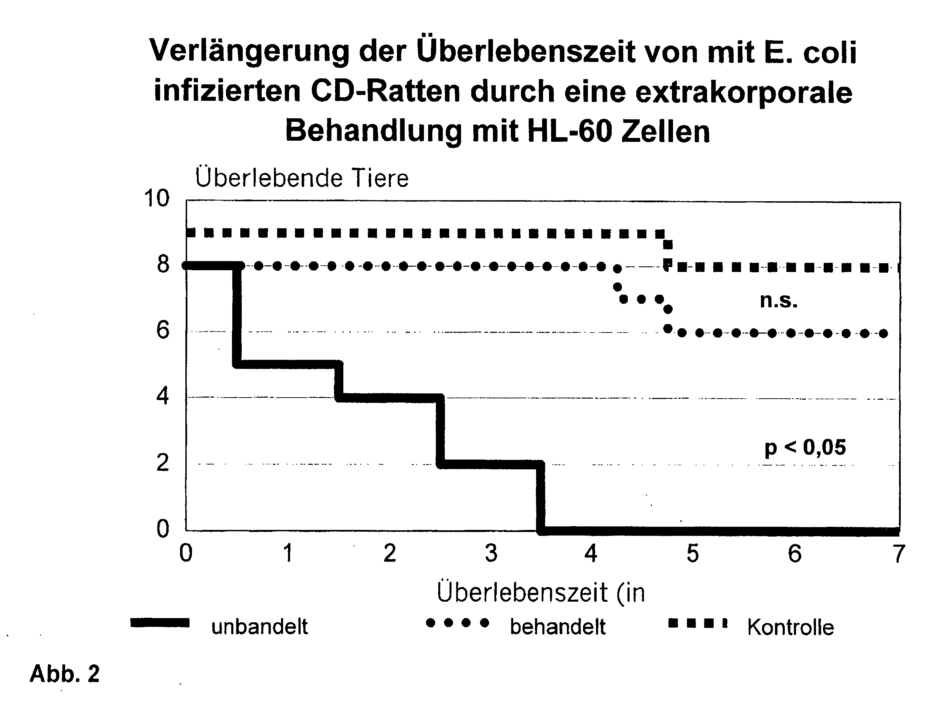

Weitere Vorteile, Merkmale und Ausgestaltungsformen der vorliegenden Erfindung werden anhand des nachfolgenden Beispiels und der dazugehörenden Abbildungen 1 und 2 deutlich.

### Beispiel

Blut von CD-Ratten, die mit Escherichia coli Bakterien behandelt worden waren, wurde durch Filtration oder Differentialzentrifugation in korpuskuläre Bestandteile, wie u.a. Blutzellen, und das Blutplasma getrennt (Plasmapherese) und das Blutplasma bzw. Blutplasmabestandteile anschließend durch den erfindungsgemäßen Bioreaktor geleitet. Als Zellen wurden in dem Bioreaktor HL60-Zellen eingesetzt, die zuvor mit Vitamin D stimuliert und differenziert worden waren. HL60-Zellen binden bekanntermaßen über spezifische Rezeptoren Zytokine, wie Interferon gamma, und Wachstumsfaktoren, wie Granulozyten-Kolonie-stimulierenden Faktor, sowie andere immunmodulierende Stoffe. Durch Adsorption dieser Stoffe an den entsprechenden Rezeptoren senkten die Zellen deren Konzentration in dem in den Bioreaktor eingebrachten Plasma und setzten andererseits jedoch auch selbst immunmodulierende Stoffe frei, wie Interleukin 6 und Tumor-Nekrose-Faktor alpha (TNF alpha). Die Konzentration von freigesetztem TNF alpha in dem Blutplasma wurde zum Zeitpunkt des Einbringens in den Bioreaktor bestimmt und als Bezugswert für die Menge des durch die stimulierten Zellen freigesetzten TNF alpha genommen. Nach 2, 6, 12 und 20 Stunden in dem Bioreaktor wurde das Plasma von den Zellen getrennt und aus dem Bioreaktor entnommen und jeweils die Konzentration an TNF alpha in dem Plasma bestimmt. Als Kontrolle wurden undifferenzierte HL60-Zellen eingesetzt. Die Ergebnisse dieses Experiments sind in Abbildung 1 grafisch dargestellt. Die Konzentration an TNF alpha in dem Plasma stieg in den ersten 6 Stunden nach der Stimulation der HL60-Zellen steil und anschließend etwas flacher, jedoch stetig an.

Das mit den stimulierten HL60-Zellen immunmodulierte Bioreaktoreluat (20 Stunden im Bioreaktor) wurde anschließend wieder mit den korpuskulären Blutbestandteilen zusammengeführt, den Ratten reinfundiert und die Überlebensrate der Ratten bestimmt. Als Kontrolle wurde die Überlebensrate von infizierten Ratten bestimmt, denen nicht behandeltes Plasma bzw. Blut reinfundiert wurde, sowie die Überlebensrate von nichtinfizierten Ratten, denen das Eluat reinfundiert wurde. Die Ergebnisse dieses Experiments sind in Abbildung 2 grafisch wiedergegeben.

Die mit Escherichia coli Bakterien infizierten Ratten überlebten wesentlich länger, wenn das Plasma in der vorbeschriebenen erfindungsgemäßen Weise behandelt worden war, und zwar fast so lange, wie die nicht infizierten Kontrollratten.

## Patentansprüche

1. Verfahren zur Erhöhung und / oder Verringerung der Konzentration immunmodulatorisch wirksamer Stoffe in Stoffgemischen oder Lösungen, die potentiell immunmodulatorisch wirksame Stoffe enthalten, bei dem man
das Stoffgemisch in einen Bioreaktor einführt und dort mit Zellen in Berührung bringt,
wobei die Zellen so ausgewählt sind, dass sie Rezeptoren für wenigstens eine Spezies aus einer Gruppe G1 immunmodulatorisch wirksamer Stoffe, deren Konzentration in dem Stoffgemisch verringert werden soll, aufweisen und in der Lage sind, diese Spezies zu adsorbieren, und / oder dass die Zellen wenigstens eine Spezies aus einer anderen Gruppe G2 immunmodulatorischer Stoffe, deren Konzentration in dem Stoffgemisch erhöht werden soll, herstellen und in der Lage sind, diese Spezies in das Stoffgemisch freizusetzen,
das Stoffgemisch anschließend von den Zellen trennt und aus dem Bioreaktor entfernt.

2. Verfahren nach Anspruch 1, **dadurch gekennzeichnet, dass** das Stoffgemisch Blut,. Blutplasma, Blutplasmabestandteile und / oder Wasser einzeln oder in Kombination und gegebenenfalls zusätzlich Eiweiße, Peptide, Kohlenhydrate, Lipide, Nukleinsäuren, Salze und / oder Mikroorganismen einzeln oder in Kombination enthält.

3. Verfahren nach einem der Ansprüche 1 oder 2, **dadurch gekennzeichnet, dass** die Gruppen G1 und / oder G2 immunmodulatorischer Stoffe Zytokine, vorzugsweise pro-inflammatorische oder anti-inflammatorische Zytokine, lösliche Zytokinrezeptoren, Zytokinrezeptorantagonisten, Wachstumsfaktoren, lösliche Adhäsionsmoleküle, Komponenten oder Spaltprodukte des Gerinnungs-, Fibrinolyse- oder Komplementsystems und reaktive Sauerstoffspezies umfassen.

4. Verfahren nach einem der Ansprüche 1 bis 3, **dadurch gekennzeichnet, dass** die Gruppen G1 und / oder G2 immunmodulatorischer Stoffe Interferon Alpha, Interferon Beta, Interferon Gamma, Interleukin 1, Interleukin 2, Interleukin 3, Interleukin 4, Interleukin 5, Interleukin 6, Interleukin 7, Interleukin 8, Interleukin 9, Interleukin 10, Interleukin 11, , Interleukin 12, Interleukin 13, Interleukin 14, Interleukin 15, Interleukin 16, Interleukin 17, Interieukin 18, Tumor-Nekrose-Faktor Alpha (TNF-alpha), Tumor-Nekrose-Faktor Beta (TNF-beta), Transformierenden Wachstumsfaktor Beta (TGF-beta), Chemokine und Chemotaxine umfassen.

5. Verfahren nach einem der Ansprüche 1 bis 4, **dadurch gekennzeichnet, dass** die Gruppen G1 und / oder G2 Bestandteile oder Produkte von Mikroorganismen, vorzugsweise Bakterien, Viren, Pilzen oder Parasiten, oder vollständige Mikroorganismen oder allergen wirkende Substanzen umfassen.

6. Verfahren nach einem der Ansprüche 1 bis 5, **dadurch gekennzeichnet, dass** die Gruppen G1 und / oder G2 Endotoxine, vorzugsweise Lipopolysaccharide, Exotoxine,. vorzugsweise Hämolysin A, B, C, D oder E, Lipoteicholsäure und Zymosan umfassen.

7. Verfahren nach einem der Ansprüche 1 bis 6, **dadurch gekennzeichnet, dass** die Gruppen G1 und / oder G2 Immunkomplexe, bestehend aus wenigstens einem Antikörper, vorzugsweise einem Immunglobulin, oder einem Antikörperanteil und wenigstens einem Antigen oder antigen wirkendem Stoff, vorzugsweise einem Hapten, umfassen.

8. Verfahren nach einem der Ansprüche 1 bis 7, **dadurch gekennzeichnet, dass** die Zellen in dem Bioreaktor Leukozyten, hämatopoietische Stammzellen, aus hämatopoietischen Stammzellen durch Differenzierung gewonnene Zellen, Hepatozyten, Endothelzellen, Nervenzellen, Mukosazellen, Epithelzellen oder andere dem Ektoderm, Mesoderm oder Endoderm entstammende Zellen oder eine Kombination davon sind.

9. Verfahren nach einem der Ansprüche 1 bis 8, **dadurch gekennzeichnet, dass** die Zellen in dem Bioreaktor Primärzellen, immortalisierte oder Tumorzellen sind.

10. Verfahren nach einem der Ansprüche 1 bis 9, **dadurch gekennzeichnet, dass** die Zellen in dem Bioreaktor Zellen humanen, tierischen, planzlichen oder mikrobiellen Ursprungs sind.

11. Verfahren nach einem der Ansprüche 1 bis 10, **dadurch gekennzeichnet, dass** die Zellen in dem Bioreaktor vor oder während der Einführung und Berührung mit dem Stoffgemisch stimuliert werden.

12. Verfahren nach einem der Ansprüche 1 bis 11, **dadurch gekennzeichnet, dass** man Temperatur, Begasung und Nährstoffzufuhr der Zeilen in dem Bioreaktor reguliert.

13. Verfahren nach einem der Ansprüche 1 bis 12, **dadurch gekennzeichnet, dass** man das Stoffgemisch nach der Berührung mit den Zellen durch eine Zellrückhalteeinrichtung, vorzugsweise einen Zellfilter oder eine Zentrifuge, von den Zellen trennt.

14. Vorrichtung zur Erhöhung und / oder Verringerung der Konzentration immunmodulatorisch wirksamer Stoffe in einem Stoffgemisch mit einem Bioreaktor mit einem Einlaß zum Einführen des Stoffgemischs und einem Auslaß zur Entnahme des Stoffgemischs, wobei in dem Bioreaktor lebende Zellen in einer Form vorhanden sind, dass das Stoffgemisch davon mit den Zellen in Berührung gebracht werden kann, und mit einer Zellrückhalteeinrichtung, die so ausgelegt ist, dass das Stoffgemisch von den Zellen trennbar und frei von Zellen aus dem Bioreaktor entnehmbar ist, **dadurch gekennzeichnet, dass** die Zellen Rezeptoren für wenigstens eine Spezies aus einer Gruppe G1 immunmodulatorisch wirksamer Stoffe, deren Konzentration in dem Stoffgemisch verringert werden soll, aufweisen und in der Lage sind, diese Spezies zu adsorbieren, und / oder dass die Zellen wenigstens eine Spezies aus einer anderen Gruppe G2 immunmodulatorischer Stoffe, deren Konzentration in dem Stoffgemisch erhöht werden soll, herstellen und in der Lage sind, diese Spezies in das Stoffgemisch freizusetzen.

15. Vorrichtung nach Anspruch 14, **dadurch gekennzeichnet, dass** das Stoffgemisch Blut, Blutplasma, Blutplasmabestandteile und / oder Wasser einzeln oder in Kombination und gegebenenfalls zusätzlich Eiweiße, Peptide, Kohlenhydrate, Lipide, Nukleinsäuren, Salze und / oder Mikroorganismen einzeln oder in Kombination enthält.

16. Vorrichtung nach einem der Ansprüche 14 oder 15, **dadurch gekennzeichnet, dass** die Gruppen G1 und / oder G2 immunmodulatorischer Stoffe Zytokine, vorzugsweise pro-inflammatorische oder anti-inflammatorische Zytokine, lösliche Zytokinrezeptoren, Zytokinrezeptorantagonisten, Wachstumsfaktoren, lösliche AdhäsionsmoleküleKomponenten oder Spaltprodukte des Gerinnungs-, Fibrinolyse- oder Komplementsystems und reaktive Sauerstoffspezies umfassen.

17. Vorrichtung nach einem der Ansprüche 14 bis 16, **dadurch gekennzeichnet, dass** die Gruppen G1 und / oder G2 immunmodulatorischer Stoffe Interferon Alpha, Interferon Beta, Interferon Gamma, Interleukin 1, Interleukin 2, Interleukin 3, Interleukin 4, Interleukin 5, Interleukin 6, Interleukin 7, Interleukin 8, Interleukin 9, Interleukin 10, Interleukin 11, , Interleukin 12, Interleukin 13, Interleukin 14, Interleukin 15, Interleukin 16, Interleukin 17, Interleukin 18, Tumor-Nekrose-Faktor Alpha (TNF-alpha), Tumor-Nekrose-Faktor Beta (TNF-beta), Transformierenden Wachstumsfaktor Beta (TGF-beta), Chemokine und Chemotaxine umfassen.

18. Vorrichtung nach einem der Ansprüche 14 bis 17, **dadurch gekennzeichnet, dass** die Gruppen G1 und / oder G2 Bestandteile oder Produkte von Mikroorganismen, vorzugsweise Bakterien, Viren, Pilzen oder Parasiten, oder vollständige Mikroorganismen oder allergen wirkende Substanzen umfassen.

19. Vorrichtung nach einem der Ansprüche 14 bis 18, **dadurch gekennzeichnet, dass** die Gruppen G1 und / oder G2 Endotoxine, vorzugsweise Lipopolysaccharide, Exotoxine, vorzugsweise Hämolysin A, B, C, D oder E, Lipoteicholsäure und Zymosan umfassen.

20. Vorrichtung nach einem der Ansprüche 14 bis 19, **dadurch gekennzeichnet, dass** die Gruppen G1 und / oder G2 Immunkomplexe, bestehend aus wenigstens einem Antikörper, vorzugsweise einem Immunglobulin, oder einem Antikörperanteil und wenigstens einem Antigen oder antigen wirkendem Stoff, vorzugsweise einem Hapten, umfassen.

21. Vorrichtung nach einem der Ansprüche 14 bis 20, **dadurch gekennzeichnet, dass** die Zellen in dem Bioreaktor Leukozyten, hämatopoietische Stammzellen, aus hämatopoietischen Stammzellen durch Differenzierung gewonnene Zellen, Hepatozyten, Endothelzellen, Nervenzellen, Mukosazellen, Epithelzellen oder andere dem Ektoderm, Mesoderm oder Endoderm entstammende Zellen oder eine Kombination davon sind.

22. Vorrichtung nach einem der Ansprüche 14 bis 21, **dadurch gekennzeichnet, dass** die Zellen in dem Bioreaktor Primärzellen, immortalisierte oder Tumorzellen sind.

23. Vorrichtung nach einem der Ansprüche 14 bis 22, **dadurch gekennzeichnet, dass** die Zellen in dem Bioreaktor Zellen humanen, tierischen, planzlichen oder mikrobiellen Ursprungs sind.

24. Vorrichtung nach einem der Ansprüche 14 bis 23, **dadurch gekennzeichnet, dass** die Zellen in dem Bioreaktor vor oder während der Einführung und Berührung mit dem Stoffgemisch stimuliert werden.

25. Vorrichtung nach einem der Ansprüche 14 bis 24, **dadurch gekennzeichnet, dass** der Bioreaktor so ausgelegt ist, dass Temperatur, Begasung und Nährstoffzufuhr der Zellen regulierbar sind.

26. Vorrichtung nach einem der Ansprüche 14 bis 25, **dadurch gekennzeichnet, dass** die Zellrückhalteeinrichtung ein Zellfilter oder eine Zentrifuge ist.

## Claims

1. A method of increasing and/or reducing the concentration of immunomodulatory-effective substances in substance mixtures or solutions which contain potentially immunomodulatory-effective substance, in which
the substance mixture is introduced into a bioreactor and there brought into contact with cells,
wherein the cells are so selected that they have receptors for at least one species from a group G1 of immunomodulatory-effective substances whose concentration in the substance mixture is to be reduced and which are in a position to adsorb said species and/or that the cells produce at least one species from another group G2 of immunomodulatory substances whose concentration in the substance mixture is to be increased and which are in a position to liberate said species into the substance mixture, and
the substance mixture is then separated from the cells and removed from the bioreactor.

2. A method according to claim 1 **characterised in that** the substance mixture contains blood, blood plasma, blood plasma constituents and/or water individually or in combination and optionally additionally albumins, peptides, carbohydrates, lipids, nucleic acids, salts and/or micro-organisms individually or in combination.

3. A method according to one of claims 1 and 2 **characterised in that** the groups G1 and/or G2 of immunomodulatory substances include cytokines, preferably pro-inflammatory or anti-inflammatory cytokines, soluble cytokine receptors, cytokine receptor antagonists, growth factors, soluble adhesion molecules, components or cleavage products of the coagulation, fibrinolysis or complement system and reactive oxygen species.

4. A method according to one of claims 1 to 3 **characterised in that** the groups G1 and/or G2 of immunomodulatory substances include interferon alpha, interferon beta, interferon gamma, interleukin-1, interleukin-2, interleukin-3, interleukin-4, interleukin-5, interleukin-6, interleukin-7, interleukin-8, interleukin-9, interleukin-10, interleukin-11, interleukin-12, interleukin-13, interleukin-14, interleukin-15, interleukin-16, interleukin-17, interleukin-18, tumour sis factor alpha (TNF-alpha), tumour necrosis factor beta (TNF-beta), transforming growth factor beta (TGF-beta), chemokines and chemotaxins.

5. A method according to one of claims I to 4 **characterised in that** the groups G1 and/or G2 include constituents or products of micro-organisms, preferably bacteria, viruses, fungi or parasites, or complete micro-organisms or allergen-effect substances.

6. A method according to one of claims 1 to 5 **characterised in that** the groups G1 and/or G2 include endotoxins, preferably lipopolysaccharides, exotoxins, preferably haemolysin A, B, C, D or E, lipoteicholic acid and zymosam.

7. A method according to one of claims 1 to 6 **characterised in that** the groups G1 and/or G2 include immunocomplexes comprising at least one antibody, preferably an immunoglobulin, or an antibody proportion and at least one antigen or antigen-effective substance, preferably a hapten.

8. A method according to one of claims 1 to 7 **characterised in that** the cells in the bioreactor arc leucocytes, haematopoietic stem cells, cells produced from haematopoieric stem cells by differentiation, hepatocytes, endothelium cells, nerve cells, mucosa cells, epithelium cells or other cells originating from the ectoderm, mesoderm or endoderm or a combination thereof.

9. , A method according to one of claims 1 to 8 **characterised in that** the cells in the bioreactor are primary cells, immortalised or tumour cells.

10. A method according to one of claims 1 to 9 **characterised in that** the cells in the bioreactor are cells of human, animal, vegetable or microbial origin.

11. A method according to one of claims 1 to 10 **characterised in that** the cells in the bioreactor are stimulated prior to or during introduction and contacting with the substance mixture.

12. A method according to one of claims 1 to 11 **characterised in that** temperature, supply of gas and nutrient feed for the cells in the bioreactor are regulated,

13. A method according to one of claims 1 to 12 **characterised in that** after contacting with the cells the substance mixture is separated from the cells by a cell retention device, preferably a cell filter or a centrifuge.

14. Apparatus for increasing and/or reducing the concentration of immunomodulatory-effective substances in a substance mixture comprising a bioreactor with an inlet for the introduction of the substance mixture and an outlet for the removal of the substance mixture, wherein living cell are present in the bioreactor in a form such that the substance mixture thereof can be brought into contact with the cells and a cell retaining device which is so designed that the substance mixture can be separated from the cells and can be removed free from cells from the bioreactor, **characterised in that** the cells have receptors for at least one species from a group G1 of immunomodulatory-effective substances whose concentrtion in the substance mixture is to be reduced and which are in a position to adsorb said species and/or that the cells produce at least one species from another group G2 of immunomodulatory substances whose concentration in the substance mixture is to be increased and which are in a position to liberate said species into the substance mixture.

15. Apparatus according to claim 14 **characterised in that** the substance mixture contains blood, blood plasma, blood plasma constituents and/or water individually or in combination and optionally additionally albumins, peptides, carbohydrates, lipids, nucleic acids, salts and/or micro-organisms individually r in combination.

16. Apparatus according to one of claims 14 and 15 **characterised in that** the groups G1 and/or G2 of immunomodulatory substances include cytokines, preferably pro-inflammatory or anti-inflammatory cytokines, soluble cytokine receptors, cytokine receptor antagonists, growth factors, soluble adhesion molecules, components or cleavage products of the coagulation, fibrinolysis or complement system and reactive oxygen species.

17. Apparatus according to one of claims 14 to 16 **characterised in that** the groups G1 and/or G2 of immunomodulatory substances include interferon alpha, interferon beta, interferon gamma, interleukin-1, interleukin-2, interleukin-3, interleukin-4, interleukin-5, interleukin-6, interleukin-7, interleukin-8, inteleukin-9, interleukin-10, interleukin-11, interleukin-12, interleukin-13, interleukin-14, interleukin-15, interleukin-16, interleukin-17, interleukin-18, tumour necrosis factor alpha (TNF-alpha), tumour necrosis factor beta (TNF-beta), transforming growth factor beta (TGF-beta), chemokines and chemotaxins.

18. Apparatus according to one of claims 14 to 17 **characterised in that** the groups G1 and/or G2 include constituents or products of micro-organisms, preferably bacteria, viruses, fungi or parasites, or complete micro-organisms or allergen-effect substances.

19. Apparatus according to one of claims 14 to 18 **characterised in that** the groups G1 and/or G2 include endotoxins, preferably lipopolysaccharides, exotoxins, preferably haemolysin A, B, C, D or E, lipoteicholic acid and zymosan.

20. Apparatus according to one of claims 14 to 19 **characterised in that** the groups G1 and/or G2 include immunocomplexes comprising at least one antibody, preferably an immunoglobulin, or an antibody proportion and at least one antigen or antigen-effective substance, preferably a hapten.

21. Apparatus according to one of claims 14 to 20 **characterised in that** the cells in the bioreactor are leucocytes, haematopoietic stem cells, cells produced from haematopoietic stem cells by differentiation, hepatocytes, endothelium cells, nerve cells, mucosa cells, epithelium cells or other cells originating from the ectoderm, mesoderm or endoderm or a combination thereof.

22. Apparatus according to one of claims 14 to 21 **characterised in that** the cells in the bioreactor are primary cells, immortalised or tumour cells.

23. Apparatus according to one of claims 14 to 22 **characterised in that** the cells in the bioreactor are cells of human, animal, vegetable or microbial origin.

24. Apparatus according to one of claims 14 to 23 **characterised in that** the cells in the bioreactor are stimulated prior to or during introduction and contacting with the substance mixture.

25. Apparatus according to one of claims 14 to 24 **characterised in that** the bioreactor is so designed that temperature, supply of gas and nutrient feed for the cells are regulatable.

26. Apparatus according to one of claims 14 to 25 **characterised in that** the cell retaining device is a cell filter or a centrifuge.

## Revendications

1. Procédé d'augmentation et/ou de réduction de la concentration en substances actives du point de vue immunomodulateur dans des solutions ou mélanges de substances qui contiennent des substances potentiellement actives du point de vue immunomodulateur, dans lequel
on introduit le mélange de matières dans un bioréacteur,
on met en contact ledit mélange avec des cellules dans ledit bioréacteur,
les cellules étant choisies de façon à comporter des récepteurs pour au moins une espèce provenant d'un groupe G1 de substances actives du point de vue immunomodulateur dont la concentration dans le mélange de substances doit être réduite, et à être en mesure d'absorber cette espèce, et/ou à fabriquer au moins une espèce provenant d'un autre groupe G2 de substances immunomodulatrices dont la concentration dans le mélange de matière doit être augmentée, et à être en mesure de libérer cette espèce dans le mélange de substances,
on sépare ensuite le mélange de substance des cellules, et on retire ledit mélange du bioréacteur.

2. Procédé selon la revendication 1, **caractérisé en ce que** le mélange de substances contient du sang, du plasma sanguin, des constituants plasmatiques sanguins et/ou de l'eau individuellement ou en combinaison et le cas échéant en plus des blancs d'oeuf, des peptides, des hydrates de carbone, des lipides, des acides nucléiques, des sels et/ou des microorganismes individuellement ou en combinaison.

3. Procédé selon l'une des revendications 1 ou 2, **caractérisé en ce que** les groupes G1 et/ou G2 de substances immunomodulatrices comportent des cytokines, de préférence des cytokines pro-inflammatoires ou anti-inflammatoires, des récepteurs cytokines solubles, des antagonistes de récepteurs cytokines, des facteurs de croissance, des molécules d'adhésion solubles, des composés ou des produits de dédoublement du système coagulant, fibrinolytique ou complémentaire et une espèce oxygénée réactive.

4. Procédé selon l'une des revendications 1 à 3, **caractérisé en ce que** les groupes G1 et/ou G2 de substances immunomodulatrices comportent l'interféron alpha, l'interféron bêta, l'interféron gamma, l'interleukine 1, l'interleukine 2, l'interleukine 3, l'interleukine 4, l'interleukine 5, l'interleukine 6, l'interleukine 7, l'interleukine 8, l'interleukine 9, l'interleukine 10, l'interleukine 11, l'interleukine 12, l'interleukine 13, l'interleukine 14, l'interleukine 15, l'interleukine 16, l'interleukine 17, l'interleukine 18, le facteur de nécrose tumorale alpha (TNF-alpha), le facteur de nécrose tumorale bêta (TNF-bêta), le facteur transformant de croissance bêta (TGF-bêta), la chemokine et chemotaxine.

5. Procédé selon l'une des revendications 1 à 4, **caractérisé en ce que** les groupes G1 et/ou G2 comportent des constituants ou produits de microorganismes, de préférence de bactéries, de virus, de champignons ou de parasites, ou des microorganismes complets ou de substances allergéniques.

6. Procédé selon l'une des revendications 1 à 5, **caractérisé en ce que** les groupes G1 et/ou G2 comportent des endotoxines, de préférence des lipopolysaccharides, des exotoxines, de préférence l'hémolysine A, B, C, D ou E, l'acide lipoteicholique et le zymosan.

7. Procédé selon l'une des revendications 1 à 6, **caractérisé en ce que** les groupes G1 et/ou G2 comportent des complexes immunologiques constitués par au moins un anticorps, de préférence une immunoglobuline, ou un pourcentage d'anticorps et au moins un antigène ou une substance antigénique, de préférence un haptène.

8. Procédé selon l'une des revendications 1 à 7, **caractérisé en ce que** les cellules dans le bioréacteur sont des leucocytes, des cellules souches hématopoïétiques, des cellules obtenues des cellules souches hématopoïétiques par différenciation, des hépatocytes, des cellules endothéliales, des cellules nerveuses, des cellules muqueuses, des cellules épithéliales ou autres cellules ectodermiques, mésodermiques ou endodermiques ou une combinaison de celles-ci.

9. Procédé selon l'une des revendications 1 à 8, **caractérisé en ce que** les cellules dans le bioréacteur sont des cellules primaires, des cellules immortalisées ou des cellules tumorales.

10. Procédé selon l'une des revendications 1 à 9, **caractérisé en ce que** les cellules dans le bioréacteur sont des cellules d'origine humaine, animale, végétale ou microbienne.

11. Procédé selon l'une des revendications 1 à 10, **caractérisé en ce que** les cellules dans le bioréacteur sont stimulées avant ou pendant l'introduction et la mise en contact avec le mélange de substances.

12. Procédé selon l'une des revendications 1 à 11, **caractérisé en ce que** l'on régule la température, le gazage et l'amenée des nutriments aux cellules dans le bioréacteur.

13. Procédé selon l'une des revendications 1 à 12, **caractérisé en ce que** l'on sépare des cellules le mélange de substances après la mise en contact avec les cellules au moyen d'un dispositif de rétention de cellules, de préférence un filtre cellulaire ou une centrifugeuse.

14. Dispositif d'augmentation et/ou de réduction de la concentration en substances actives du point de vue immunomodulateur dans un mélange de substances, lequel dispositif comporte un bioréacteur doté d'une entrée destinée à introduire le mélange de substances et d'une sortie destinée à retirer le mélange de substances, les cellules vivantes se trouvant dans le bioréacteur sous une forme telle que le mélange de substances peut être mis en contact avec les cellules, et un dispositif de rétention de cellules qui est étudié pour que le mélange de cellules puisse être séparé des cellules et être retiré du bioréacteur en étant libéré des cellules,
**caractérisé en ce que** les cellules comportent des récepteurs pour au moins une espèce provenant d'un groupe G1 de substances actives du point de vue immunomodulateur dont la concentration dans le mélange de substances doit être réduite et sont en mesure d'absorber cette espèce et/ou **en ce que** les cellules fabriquent au moins une espèce d'un autre groupe G2 de substances immunomodulatrices dont la concentration dans le mélange de substances doit être augmentée, et sont en mesure de libérer cette espèce dans le mélange de substances.

15. Dispositif selon la revendication 14, **caractérisé en ce que** le mélange de substances contient du sang, du plasma sanguin, des constituants plasmatiques sanguins et/ou de l'eau individuellement ou en combinaison et le cas échéant en plus des blancs d'oeuf, des peptides, des hydrates de carbone, des lipides, des acides nucléiques, des sels et/ou des microorganismes individuellement ou en combinaison.

16. Dispositif selon l'une des revendications 14 ou 15, **caractérisé en ce que** les groupes G1 et/ou G2 de substances immunomodulatrices comportent des cytokines, de préférence des cytokines pro-inflammatoires ou anti-inflammatoires, des récepteurs cytokines solubles, des antagonistes de récepteurs cytokines, des facteurs de croissance, des molécules d'adhésion solubles, des composés ou des produits de dédoublement du système coagulant, fibrinolytique ou complémentaire et une espèce oxygénée réactive.

17. Dispositif selon l'une des revendications 14 à 16, **caractérisé en ce que** les groupes G1 et/ou G2 de substances immunomodulatrices comportent l'interféron alpha, l'interféron bêta, l'interféron gamma, l'interleukine 1, l'interleukine 2, l'interleukine 3, l'interleukine 4, l'interleukine 5, l'interleukine 6, l'interleukine 7, l'interleukine 8, l'interleukine 9, l'interleukine 10, l'interleukine 11, l'interleukine 12, l'interleukine 13, l'interleukine 14, l'interleukine 15, l'interleukine 16, l'interleukine 17, l'interleukine 18, le facteur de nécrose tumorale alpha (TNF-alpha), le facteur de nécrose tumorale bêta (TNF-bêta), le facteur transformant de croissance bêta (TGF-bêta), la chemokine et chemotaxine.

18. Procédé selon l'une des revendications 14 à 17, **caractérisé en ce que** les groupes G1 et/ou G2 comportent des constituants ou produits de microorganismes, de préférence de bactéries, de virus, de champignons ou de parasites, ou des microorganismes complets ou de substances allergéniques.

19. Procédé selon l'une des revendications 14 à 18, **caractérisé en ce que** les groupes G1 et/ou G2 comportent des endotoxines, de préférence des lipopolysaccharides, des exotoxines, de préférence l'hémolysine A, B, C, D ou E, l'acide lipoteicholique et le zymosan.

20. Procédé selon l'une des revendications 14 à 19, **caractérisé en ce que** les groupes G1 et/ou G2 comportent des complexes immunologiques constitués par au moins un anticorps, de préférence une immunoglobuline, ou un pourcentage d'anticorps et au moins un antigène ou une substance antigénique, de préférence un haptène.

21. Procédé selon l'une des revendications 14 à 20, **caractérisé en ce que** les cellules dans le bioréacteur sont des leucocytes, des cellules souches hématopoïétiques, des cellules obtenues des cellules souches hématopoïétiques par différenciation, des hépatocytes, des cellules endothéliales, des cellules nerveuses, des cellules muqueuses, des cellules épithéliales ou autres cellules ectodermiques, mésodermiques ou endodermiques ou une combinaison de celles-ci.

22. Procédé selon l'une des revendications 14 à 21, **caractérisé en ce que** les cellules dans le bioréacteur sont des cellules primaires, des cellules immortalisées ou des cellules tumorales.

23. Procédé selon l'une des revendications 14 à 22, **caractérisé en ce que** les cellules dans le bioréacteur sont des cellules d'origine humaine, animale, végétale ou microbienne.

24. Procédé selon l'une des revendications 14 à 23, **caractérisé en ce que** les cellules dans le bioréacteur sont stimulées avant ou pendant l'introduction et la mise en contact avec le mélange de substances.

25. Procédé selon l'une des revendications 14 à 24, **caractérisé en ce que** l'on régule la température, le gazage et l'amenée des nutriments aux cellules dans le bioréacteur.

26. Procédé selon l'une des revendications 14 à 25, **caractérisé en ce que** l'on sépare des cellules le mélange de substances après la mise en contact avec les cellules au moyen d'un dispositif de rétention de cellules, de préférence un filtre cellulaire ou une centrifugeuse.
